# EUROPEAN PATENT SPECIFICATION

(11) **EP 3 203 228 B1**
(45) Date of publication and mention of the grant of the patent: **26.08.2020**
(21) Application number: 14903260.9
(22) Date of filing: 30.09.2014
(51) Int. Cl.: G01N 33/68, G16H 50/30

(54) **CHRONIC HEART DISEASE PATIENT SPECIFIC BIOMARKER COMPOSITION AND USE THEREOF**
SPEZIFISCHE BIOMARKERZUSAMMENSETZUNG FÜR PATIENTEN MIT CHRONISCHER HERZKRANKHEIT UND VERWENDUNG DAVON
COMPOSITION DE BIOMARQUEUR SPÉCIFIQUE D'UN PATIENT ATTEINT D'UNE MALADIE CARDIAQUE CHRONIQUE ET UTILISATION ASSOCIÉE

(43) Date of publication of application: 09.08.2017
(73) Proprietor: BGI Shenzhen Co., Limited, Shenzhen, Guangdong 518083 (CN); BGI Shenzhen, Shenzhen, Guangdong 518083 (CN)
(72) Inventor: FENG, Qiang, Shenzhen, Guangdong 518083 (CN); LIU, Zhipeng, Shenzhen, Guangdong 518083 (CN); MENG, Nan, Shenzhen, Guangdong 518083 (CN); WANG, Jun, Shenzhen, Guangdong 518083 (CN)
(74) Representative: Inspicos P/S
(86) International application number: PCT/CN2014/087852
(87) International publication number: WO 2016/049829

(56) References cited:
- WO-A1-2010/012306
- WO-A2-2008/118413
- WO-A2-2010/138899
- WO-A2-2013/171586
- CN-A- 101 769 910
- CN-A- 102 323 351
- US-B1- 7 459 286
- HALEEM, J.I. ET AL.: 'Detection of Bladder Cancer in Human Urine by Metabolomic Profiling Using High Performance Liquid Chromatography/Mass Spectrometry' THE JOURNAL OF UROLOGY vol. 179, no. 6, 23 April 2008, pages 2422 - 2426, XP022650280

## Description

### Technical Field

The present invention relates to a disease-specific metabolite profile, and particularly to a biomarker composition obtained by screening from urine-specific metabolite profiles of coronary heart disease subjects. The present invention also relates to a use of the biomarker compositions in risk assessment, diagnosis, early diagnosis, or pathological staging of coronary heart disease, and to a method for risk assessment, diagnosis, early diagnosis, or pathological staging of coronary heart disease.

### Background Art

Coronary artery heart disease (CAHD), also known as ischemic heart disease, or coronary heart disease for short, is one of the most common heart diseases, referring to dysfunctions and/or organic pathologic changes of cardiac muscles caused by coronary artery stenosis or insufficient blood supply, thus it is also called as ischemic heart disease (IHD). In 2012, it is the first cause of death in the world^{[1]}, and one of the major reasons for hospitalization^{[2]}. Coronary heart disease may occur at any age, even in children, but the major age of onset is middle age, and its incidence increases with age. Nearly 17 million people die from atherosclerotic heart diseases every year in the world, and it is estimated that there is an increase of 50% in deaths by 2020, reaching 25 million per year, accounting for 1/3 of deaths in the world. In China, there are 2.5 million people die from cardiovascular diseases per year; the new myocardial infarctions occur in 500,000 people per year; the occurrence of coronary heart disease has significant regional differences, that is, it is generally higher in the northern cities than the southern cities; there are also significant gender differences, that is, the ratio of men to women is 2-5: 1. The data show that there are also similar differences in distribution of coronary heart disease in patients in the world^{[3]}. At present, the diagnosis of coronary heart disease still lacks a uniform standard, and the existing diagnostic methods such as electrocardiogram, electrocardiogram stress test, dynamic electrocardiogram, radionuclide myocardial imaging, echocardiography, hematological examination, coronary CT, coronary angiography and intravascular imaging techniques all have some shortcomings. For example, the observation of symptoms, echocardiography and so on have strong subjectivity, the coronary CT, coronary angiography and intravascular imaging techniques are invasive diagnosis which cause additional pains in patients. The diagnosis using the single markers that have been found in blood has disadvantages such as poor sensitivity and specificity, and high false positive rate. It is of great significance to develop a noninvasive, specific and accurate method for the diagnosis of coronary heart disease^{[4,5]}.

Metabolomics is a systematic biology discipline developed after genomics and proteomics to study the species, quantities and variations of endogenous metabolites in a subject after affections of internal or external factors. Metabolomics is to analyze the whole metabolic profile of an organism, and to explore the corresponding relationships between metabolites and physiological and pathological changes, so as to provide a basis for the diagnosis of diseases. Therefore, it is of great significance to screen metabolic markers associated with coronary heart disease, in particular to use a combination of multiple metabolic markers, for the metabolomics research, clinical diagnosis and treatment of coronary heart disease.

US 7459286 discloses methods for characterizing the near term risk of experiencing a major adverse cardiac event in a patient presenting with chest pain by use of diagnostic biomarkers in body samples.

WO 2010/138899 disclosees markers and methods for determining whether a subject, particularly a human subject, has or is at risk of developing, a disease such as cardiovascular disease.

WO 2010/012306 discloses kidney biomarkers useful in the monitoring of renal function and in the prognosis and diagnosis of renal dysfunctions.

WO 2008/118413 discloses markers for detecting coronary artery disease indicative of a patient being at risk of having or developing coronary artery disease.

### Contents of the Invention

Aiming at the shortcomings such as trauma and invasion of the existing diagnostic methods for coronary artery diseases, the problem to be solved by the present invention is to provide a biomarker combination (i.e., a biomarker composition) that can be used for the diagnosis and risk assessment of coronary heart disease, and a method for diagnosis and risk assessment of coronary heart disease.

In the present invention, liquid chromatography-mass spectrometry is used for analyzing the metabolite profiles of plasma samples of the coronary heart disease group and the control group, and pattern recognition is used for analyzing and comparing the metabolite profiles of the coronary heart disease group and the control group, so as to determine specific liquid chromatography-mass spectrometry data and corresponding specific biomarkers, which provide a basis for the subsequent theoretical research and clinical diagnosis.

The first aspect of the present invention relates to a method for risk assessment, diagnosis, early diagnosis or pathological staging of coronary heart disease, comprising a step of determining content of each biomarker of a biomarker composition in a urine sample of a subject;
wherein, the biomarker composition comprises at least Biomarkers 1 to 3 of the following Biomarkers 1 to 8:
Biomarker 1, which has a mass-to-charge ratio of 356.07 ± 0.4amu, and a retention time of 606.57 ± 60s;
Biomarker 2, which has a mass-to-charge ratio of 284.18 ± 0.4amu, and a retention time of 538.89 ± 60s;
Biomarker 3, which has a mass-to-charge ratio of 445.06 ± 0.4amu, and a retention time of 494.89 ± 60s;
Biomarker 4, which has a mass-to-charge ratio of 268.19 ± 0.4amu, and a retention time of 589.52 ± 60s;
Biomarker 5, which has a mass-to-charge ratio of 342.03 ± 0.4amu, and a retention time of 625.52 ± 60s;
Biomarker 6, which has a mass-to-charge ratio of 324.0459 ± 0.4amu, and a retention time of 612.39 ± 60s;
Biomarker 7, which has a mass-to-charge ratio of 324.0457 ± 0.4amu, and a retention time of 652.06 ± 60s; and
Biomarker 8, which has a mass-to-charge ratio of 307.02 ± 0.4amu, and a retention time of 607.78 ± 60s;
wherein a liquid chromatography-mass spectrometry method is used for determining content of each biomarker of the biomarker composition in a urine sample of a subect;
wherein a C18 column and an elution solvent of 0.1% (v/v) formic acid in acetonitrile solution is used in the liquid chromatography-mass spectrometry method;.
wherein chromatographic conditions are as follows:
Column: C18 column: mobile phase A: 0.1% formic acid aqueous solution, mobile phase B: 0.1% formic acid in acetonitrile solution: gradient elution program: 0 ∼ 3 min, 5% B. 3 ∼ 36 min, 5% ∼ 80% B. 36 ∼ 40 min, 80% ∼ 100% B. 40 ∼ 45 min, 100% B. 45 ∼ 50 min, 100% ∼ 5% B. 50 ∼ 60 min, 5% B; flow rate: 0.2 mL / min; injection volume: 20 µL.

In one embodiment of the present invention, the characteristics of the above eight biomarkers are shown in Table 1.

In one embodiment of the present invention, the biomarker composition comprises at least Biomarkers 1 to 3; optionally, further comprises one or more, for example one, two, three, four or five, of Biomarkers 4 to 8.

In one embodiment of the present invention, the biomarker composition comprises Biomarkers 1 to 8.

In one instance of the present disclosure, the biomarker composition comprises Biomarkers 2, 4 to 8. Disclosed herein is also a reagent composition, comprising a reagent for detecting the biomarker composition according to the first aspect of the present invention.

The reagent for detecting the biomarker is, for example, a ligand such as an antibody that can bind to the biomarker; optionally, the reagent for detection may also have a detectable label. The reagent composition is a combination of all detection reagents.

Disclosed herein is further the use of the biomarker composition according to the first aspect and/or the reagent composition according to the second aspect of the present invention in manufacture of a kit, in which the kit is used for risk assessment, diagnosis, early diagnosis or pathological staging of coronary heart disease.

In an instance the kit further comprises training set data for the contents of the biomarker composition according to the first aspect of the present invention in a coronary heart disease subject and a normal subject.

In one embodiment, the training set data are shown in Table 2.

The present invention also relates to a method for risk assessment, diagnosis, early diagnosis or pathological staging of coronary heart disease, comprising a step of determining content of each biomarker of the biomarker composition according to the first aspect of the present invention in a sample (i.e., urine) of a subject, as defined in the claims.

In the present invention, a liquid chromatography-mass spectrometry method is used for determining the content of each biomarker of the biomarker composition according to the first aspect of the present invention in a sample (i.e. urine) of the subject.

In one embodiment of the present invention, the method further comprises a step of establishing a training set for contents of the biomarker composition according to the first aspect of the present invention in samples (e.g., urine) of a coronary heart disease subject and a normal subject (control group).

In one embodiment of the present invention, the training set is established by using a multivariate statistical classification model (e.g., a random forest model).

In one embodiment of the present invention, the training set comprises data as shown in Table 2.

In one embodiment of the present invention, the method further comprises a step of comparing the content of each biomarker of the biomarker composition according to the first aspect of the present invention in a sample (e.g., urine) of the subject to the data of training set of the coronary heart disease subject and the normal subject.

In one embodiment of the present invention, the training set is established by using a multivariate statistical classification model (e.g., a random forest model).

In one embodiment of the present invention, the training set comprises data as shown in Table 2.

In one embodiment of the present invention, the step of comparing is carried out by using a receiver operating characteristic curve (ROC).

In one embodiment of the present invention, the result of the comparing step is interpreted by a method comprising: if a subject is assumed to be a non-coronary heart disease subject, and his probability of non-coronary heart disease diagnosed by ROC is less than 0.5 or his probability of coronary heart disease diagnosed by ROC is greater than 0.5, the subject is determined to have a high probability or a higher risk of coronary heart disease, or is diagnosed as a patient with coronary heart disease.

In a particular embodiment of the present invention, the method comprises the steps of:
1) determining the content of each biomarker of the biomarker composition according to the first aspect of the present invention in urine of a subject by means of liquid chromatography-mass spectrometry;
2) determining the content of each biomarker of the biomarker composition according to the first aspect of the present invention in urine of a coronary heart disease subject and a normal subject by means of liquid chromatography-mass spectrometry, and establishing a training set (for example, as shown in Table 2) for the content of the biomarker composition by using a random forest model;
3) comparing the content of each biomarker of the biomarker composition according to the first aspect of the present invention in urine of the subject to the data of the training set of the biomarker composition of the coronary heart disease subject and the normal subject by using ROC curves;
4) if a subject is assumed to be a non-coronary heart disease subject, and his probability of non-coronary heart disease diagnosed by ROC is less than 0.5 or his probability of coronary heart disease diagnosed by ROC is greater than 0.5, the subject is determined to have a high probability or a higher risk of coronary heart disease, or is diagnosed as a Patient with coronary heart disease.

Disclosed herein is further the biomarker composition disclosed herein, which is used in risk assessment, diagnosis, early diagnosis or pathological staging of coronary heart disease.

In the present invention, a liquid chromatography-mass spectrometry method is used for determining the content of each biomarker of the biomarker composition according to the first aspect of the present invention in a sample (i.e. urine) of the subject.

In one embodiment of the present invention, it further comprises a step of establishing a training set for content of each biomarker of the biomarker composition according to the first aspect of the present invention of a coronary heart disease subject and a normal subject.

In one embodiment of the present invention, the training set is established by using a multivariate statistical classification model (e.g., a random forest model).

In one embodiment of the present invention, the training set comprises data as shown in Table 2.

In one embodiment of the present invention, it further comprises a step of comparing the content of each biomarker of the biomarker composition according to the first aspect of the present invention in a sample (e.g., urine) of the subject to the data of training set for the biomarker composition of the coronary heart disease subject and the normal subject.

In one embodiment of the present invention, the training set is established by using a multivariate statistical classification model (e.g., a random forest model).

In one embodiment of the present invention, the training set comprises data as shown in Table 2.

In one embodiment of the present invention, the comparing is performed by using a receiver operating characteristic curve for comparison.

In one embodiment of the present invention, the result of the comparing step is interpreted by a method comprising: if a subject is assumed to be a non-coronary heart disease subject, and his probability of non-coronary heart disease diagnosed by ROC is less than 0.5 or his probability of coronary heart disease diagnosed by ROC is greater than 0.5, the subject is determined to have a high probability or a higher risk of coronary heart disease, or is diagnosed as a patient with coronary heart disease.

In an instance of the disclosure, the content of each biomarker in the biomarker composition and the data of content of each biomarker in the training set are obtained by the following steps:
(1) collection and treatment of samples: an urine sample is collected from a clinical patient or a model animal;
   the sample is subjected to process, such as liquid-liquid extraction using an organic solvent, wherein the organic solvent includes, but is not limited to, ethyl acetate, chloroform, diethyl ether, n-butanol, petroleum ether, dichloromethane, acetonitrile, etc.; or protein precipitation, wherein the protein precipitation comprising precipitation of adding an organic solvent (such as methanol, ethanol, acetone, acetonitrile, isopropyl alcohol), various acid, alkali or salt precipitation, heating precipitation, filtration/ultrafiltration, solid-phase extraction, centrifugation, in single or comprehensive manner;
   the sample is dried or not dried, and then dissolved in an organic solvent (e.g., methanol, acetonitrile, isopropanol, chloroform, etc., preferably methanol, acetonitrile) or water (in single or combination, with or without salt);
   and then the sample is not derivatized or derivatized with a reagent (e.g., trimethylsilane, ethyl chloroformate, N-methyltrimethylsilyl trifluoroacetamide, etc.).
(2) liquid chromatography-mass spectrometry (HPLC-MS): a metabolite profile of urine is obtained by liquid chromatography and mass spectrometry, the metabolite profile is processed to obtain data of each peak such as peak height or peak area (peak intensity), mass-to-charge ratio and retention time, in which the peak area represents biomarker content.

In a particular instance of the present disclosure, the treatment in step (1) comprises the following step: the sample is subjected to liquid-liquid extraction with an organic solvent; or to protein precipitation; the sample is dried or not dried, and then dissolved in single or combination of organic solvents or water, the water is free of salt or contains a salt, and the salt comprises sodium chloride, phosphate, carbonate and the like; the sample is not derivatized or derivatized with a reagent.

In a specific instance of the present disclosure, in the liquid-liquid extraction with organic solvent in step (1), the organic solvent includes, but is not limited to, ethyl acetate, chloroform, diethyl ether, n-butanol, petroleum ether, dichloromethane, acetonitrile.

In a particular instance of the present disclosure, the protein precipitation in step (1) comprises, but is not limited to, precipitation of adding an organic solvent, or various acid, alkali or salt precipitation, heating precipitation, filtration/ultrafiltration, solid phase extraction, centrifugation in single or combination manner, in which the organic solvent comprises methanol, ethanol, acetone, acetonitrile, isopropanol.

In a specific instance of the present disclosure, step (1) preferably comprises performing the treatment by using a protein precipitation method, preferably a protein precipitation using ethanol.

In a specific instance of the present disclosure, in step (1), the sample is dried or not dried, and then dissolved in an organic solvent or water; the organic solvent includes methanol, acetonitrile, isopropanol, chloroform, preferably methanol, acetonitrile.

In a specific instance of the present disclosure, in step (1), the sample is derivatized with a reagent, the reagent comprises trimethylsilane, ethyl chloroformate, N-methyltrimethylsilyl trifluoroacetamide.

In a specific instance of the present disclosure, in step (2), the metabolite profile is processed to obtain raw data, the raw data are preferably data of peak height or peak area, as well as mass number and retention time of each peak.

In a specific instance of the present disclosure, in step (2), the raw data are subjected to peak detection and peak matching, the peak detection and the peak matching are preferably performed by using XCMS software.

The mass spectrometry types are roughly divided into four types including ion trap, quadrupole, electrostatic field orbital ion trap, and time-of-flight mass spectrometries, and the mass deviations of these four types are 0.2 amu, 0.4 amu, 3 ppm and 5 ppm, respectively. The experimental results in the present invention are obtained by ion trap analysis, and therefore suitable for all mass spectrometric instruments using ion trap and quadrupole as mass analyzers, including Thermo Fisher's LTQ Orbitrap Velos, Fusion, Elite et al., Waters' TQS, TQD, etc. , AB Sciex 5500, 4500, 6500, etc., Agilent's 6100, 6490, Bruker's amaZon speed ETD and so on.

In an instance of the present disclosure, the content of biomarker is expressed by peak area (peak intensity) of mass spectrum.

In the present invention, the mass-to-charge ratio and the retention time have the meanings in the art.

It is well known to those skilled in the art that the atomic mass unit and retention time of each biomarker of the biomarker composition of the present invention will fluctuate within certain ranges when different liquid chromatography-mass spectrometry devices and different detection methods are employed; wherein the atomic mass unit may fluctuate within a range of ± 0.4 amu, for example ± 0.2 amu, for example ± 0.1 amu, and the retention time may fluctuate within a range of ± 60 s, for example ± 45 s, for example ± 30 s, for example ± 15 s.

In the present invention, the methods of using the random forest model and the ROC curves are well known in the art (see the references [7] and [8]), and those skilled in the art can set and adjust parameters according to specific situations.

In the present invention, the training set and test set have the meanings well known in the art. In an embodiment of the invention, the training set refers to a data set of contents for biomarkers in samples of coronary heart disease subjects and normal subjects having given numbers. The test set is a set of data used to test the performance of the training set.

In an embodiment of the present invention, a training set of biomarkers of coronary heart disease subjects and normal subjects is constructed, and the content values of biomarkers of test samples are evaluated using the training set as basis.

In an embodiment of the present invention, the training set comprises data as shown in Table 2. In the present invention, the subject may be a human or a model animal.

In the present invention, the unit of mass-to-charge ratio is amu, amu refers to atomic mass unit, also known as Dalton (Da, D), which is a unit used to measure atomic or molecular mass, and is defined as 1/12 of atomic mass of C-12.

In the present disclosure, one or more of the biomarkers may be used for risk assessment, diagnosis or pathological staging, etc., of coronary heart disease, preferably at least three of them, i.e., Biomarkers 1 to 3, are used for evaluation, or all of the eight biomarkers (i.e., Biomarkers 1 to 8) are used for evaluation, so as to obtain desired sensitivity and specificity.

Those skilled in the art would understand that when sample size is further expanded, the normal content value interval (absolute value) of each biomarker in a sample can be obtained using sample detection and calculation methods known in the art. In this way, when the content of the biomarker is detected by methods other than mass spectrometry (for example, by using an antibody and an ELISA method), the absolute value of the detected biomarker content can be compared with the normal content value, optionally, risk assessment, diagnosis or pathological staging, etc., of coronary heart disease can also be achieved in combintion with statistical methods.

Without being bound by any theory, the inventors have pointed out that these biomarkers are endogenous compounds present in human body. The metabolite profile of urine of a subject is analyzed by the method of the present invention, and the mass value and the retention time in the metabolite profile indicate the presence and the corresponding position of the corresponding biomarker in the metabolite profile. At the same time, the biomarkers of coronary heart disease population exhibit certain content ranges in their metabolite profiles.

Endogenous small molecules in body are the basis of life activities, and changes of disease states and body functions will inevitably lead to changes of metabolism of the endogenous small molecules in the body. The present invention shows that there are significant differences in urine metabolite profiles between the coronary heart disease group and the control group. In the present invention, a plurality of relevant biomarkers are obtained through comparison and analysis of metabolite profiles of the coronary heart disease group and the control group, which can be used in combintion with high quality data of metabolite profiles of biomarkers of coronary heart disease population and normal population as the training set to accurately perform risk assessment, early diagnosis and pathological staging of coronary heart disease. Compared with the commonly used diagnostic methods, this method has advantages of noninvasion, convenience and rapid, and has high sensitivity and good specificity.

### Brief Description of the Drawings

Fig.1 shows total ion chromatograms of mass spectrometry for coronary heart disease group (a) and normal group (b).
Fig.2 shows PLS-DA score plots, in which prisms (white) represent normal group, triangles (black) represent coronary heart disease group.
Fig.3 shows a loading-plot of principal components, in which triangles (black) represent variables with VIP value greater than 1.
Fig.4 shows a Volcano-plot, in which differential metabolites are located above the horizontal dotted line, wherein the materials (black triangles) on the ambilateral sides of the two vertical dashed lines are metabolites with fold-change greater than 1.2 and Q-value less than 0.05, and the materials (gray spheres) between the two vertical dashed lines are metabolites with fold-change less than 0.8 and Q-value less than 0.05.
Fig.5 shows S-plot, in which prisms (black) represent variables with VIP greater than 1.
Fig.6 shows ROC diagram of random forest model (Random forest model), in which Training ROC is based on the training set, AUC = 1; and Test ROC is based on the test set, AUC = 0.9449.
Fig.7 shows ROC test set diagram in which mass-to-charge ratios 356.07 and 445.06 are randomly removed from the training set, AUC = 0.9289.
Fig.8 shows diagram for random combinations of 8 potential markers, in which the left side of the vertical line mark gives 3 markers that need to be tested at least.

### Specific Models for Carrying Out the Invention

While the embodiments of the present invention will be described in detail with reference to the following examples, it will be understood by those skilled in the art that the following examples are intended to be illustrative of the invention and are not to be taken as limiting the scope of the invention. In the examples, when specific conditions are not given, conventional conditions or conditions recommended by the manufacturer are employed. The used reagents or instruments which manufacturers are not given are all conventional products commercially available in the markets.

The urine samples of coronary heart disease and normal subjects in the present invention are from the Guangdong General Hospital.

### Example 1

1.1 Collection of samples: morning urine samples of volunteers were collected, immediately placed and stored in -80°C low temperature refrigerator. A total of 52 urine samples were collected from the normal group and 40 urine samples were collected from the coronary heart disease group.

1.2 Treatment of samples: frozen samples were thawed at room temperature, 500 µL of each urine sample was taken and placed in 2.0 mL centrifuge tube, added with 500 µL of methanol for dilution, centrifuged at 10000 rpm for 5 min, for standby.

1.3 Analysis by liquid chromatography-mass spectrometry
Instrument and equipment
HPLC-MS-LTQ Orbitrap Discovery (Thermo, Germany)
Chromatographic conditions
Column: C18 column (150 mm × 2.1 mm, 5 µm); Solvent A was 0.1% (v/v) formic acid/water, and solvent B was 0.1% (v/v) formic acid/methanol.; gradient elution program: 0 ∼ 3 min, 5% B, 3 ∼ 36 min, 5% ∼ 80% B, 36 ∼ 40 min, 80% ∼ 100% B, 40 ∼ 45 min, 100% B, 45 ∼ 50 min, 100% ∼ 5% B, 50 ∼ 60 min, 5% B; flow rate: 0.2 mL / min; injection volume: 20 µL.

### Mass spectrometry conditions

ESI ion source, positive ion mode for data acquisition, the mass scanning range was 50 ∼ 1000 mass-to-charge (m/z). Ion source parameters ESI: sheath gas was 10, auxiliary air was 5, capillary temperature was 350°C, spray voltage was 4.5KV.

### 1.4 Data processing

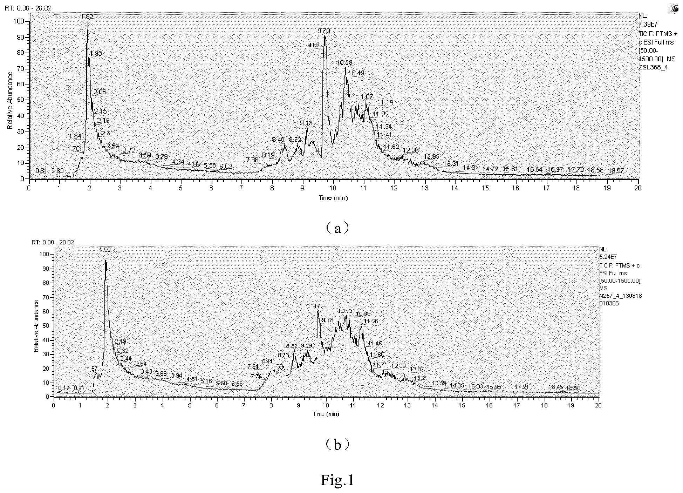

XCMS software (e.g., http://metlin.scripps.edu/xcms/) was used for peak detection and peak matching of raw data; and R software using PLS-DA (partial least squares - discriminant analysis) was used for pattern recognition analysis of differential variables of the metabolite profile of coronary heart disease group (Fig. 1a) and the metabolite profile of normal group (Fig.1b), so as to establish PLS-DA mathematical model.

### 1.5 Comparison and determination of characteristic metabolite profiles

The urine metabolite profile of coronary heart disease patients (Fig. 1) was established by comparing the urine metabolite profiles of the normal group and the coronary heart disease group. The results showed that there were significant differences in the urine metabolite profiles between the normal group and the coronary heart disease group.

### Example 2

2.1 Sample collection: morning urine samples of volunteers were collected, immediately placed and stored in -80°C low temperature refrigerator. A total of 52 urine samples were collected from the normal group and 40 urine samples were collected from the coronary heart disease group.

2.2 Sample treatment: frozen samples were thawed at room temperature, 500 µL of each urine sample was taken and placed in 2.0 mL centrifuge tube, added with 500 µL of methanol for dilution, centrifuged at 10000 rpm for 5 min, for standby.
2.3 Analysis by liquid chromatography-mass spectrometry
Instrument and equipment
HPLC-MS-LTQ Orbitrap Discovery (Thermo, Germany)
Chromatographic conditions
Column: C18 column (150 mm × 2.1 mm, 5 µm); mobile phase A: 0.1% formic acid aqueous solution, mobile phase B: 0.1% formic acid in acetonitrile solution; gradient elution program: 0 ∼ 3 min, 5% B, 3 ∼ 36 min, 5% ∼ 80% B, 36 ∼ 40 min, 80% ∼ 100% B, 40 ∼ 45 min, 100% B, 45 ∼ 50 min, 100% ∼ 5% B, 50 ∼ 60 min, 5% B; flow rate: 0.2 mL / min; injection volume: 20 µL.

### Mass spectrometry conditions

ESI ion source, positive ion mode for data acquisition, scanning mass m/z 50 ∼ 1000. Ion source parameters ESI: sheath gas was 10, auxiliary air was 5, capillary temperature was 350°C, cone hole voltage was 4.5KV.

### 2.4 Data processing

XCMS software was used for relevant pretreatment of raw data to obtain a two-dimensional matrix data, and wilcox-test was used to statistically determine significant differences of peaks of metabolites; and PLS-DA (partial least squares - discriminant analysis) was used for pattern recognition analysis of differential variables of the metabolite profile of coronary heart disease group (Fig.1a) and the metabolite profile of normal group (Fig. 1b), and potential biomarkers were screened out by VIP, Volcano-plot and S-plot in combination.

### 2.5 Metabolic profile analysis and potential biomarkers

### 2.5.1 Orthogonal partial least squares discriminant analysis (PLS-DA)

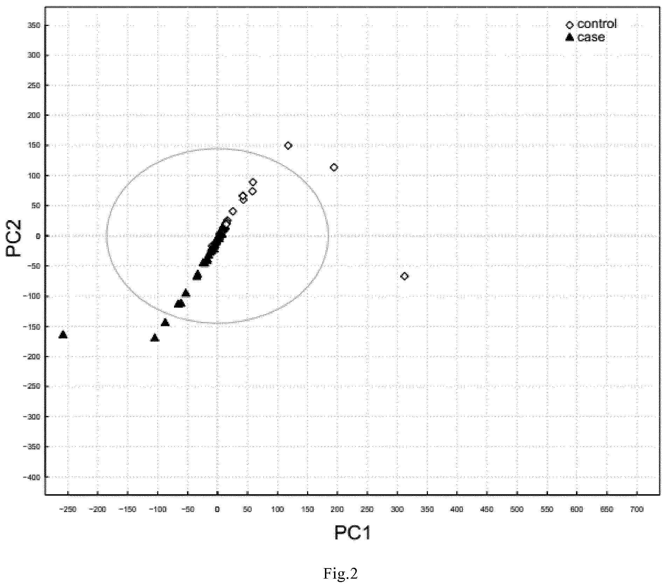

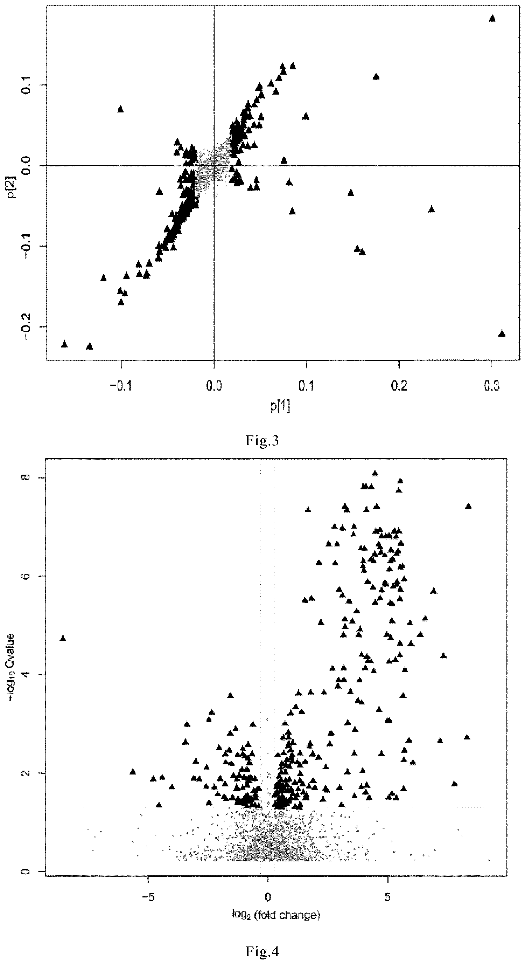

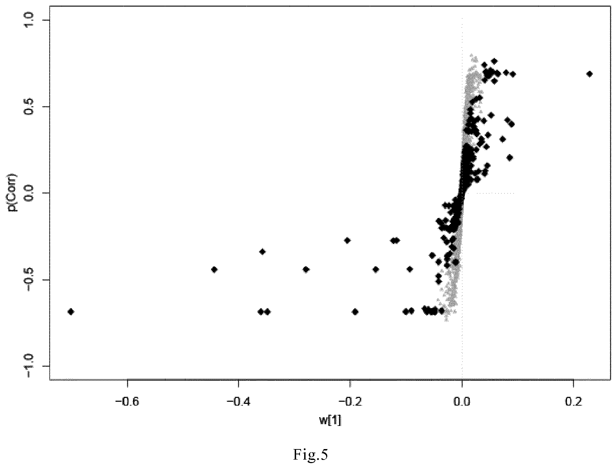

PLS-DA method was used to distinguish the normal group and the coronary heart disease group, and potential markers were further screened by VIP values (loading plot of principal component analysis) (Fig. 3), Volcano-plot (Fig. 4) and S-plot (Fig. 5). It was shown in Fig.3 and Fig.4 that there were significant different metabolites in the normal group and coronary heart disease group. As shown in Fig.5, each point in the S-plot represented a variable, and the S-plot graph showed the relevance of the variable to the model. The black prism-tagged variable was a variable with VIP greater than 1, which had a large deviation and a good correlation with the model (see Fig.2 and Fig.5).

### 2.5.2 Potential biomarkers

The potential markers were screened according to the VIP values of the PLS-DA model for pattern cognition. The variables with VIP values greater than 1 were extracted from the PLS-DA model, and variables with large deviation and relevance were further selected according to load chart, Volcano-plot and S-plot, and 8 potential biomarkers were obtained by further combining variables with P value of less than 0.05 and Q value of less than 0.05, which were shown in Table 1.

**Table 1: Potential biomarkers**

| Mass-to-ch arge ratio (amu) | Retention time, Rt (sec) | Ratio (normal group/coronar y heart disease group) | P value | Q value | VIP value |
|---|---|---|---|---|---|
| 356.07 | 606.57 | 0.05 | 2.75E-11 | 1.53E-08 | 1.26 |
| 284.18 | 538.89 | 0.02 | 1.83E-05 | 2.72E-04 | 1.45 |
| 445.06 | 494.89 | 0.02 | 1.44E-11 | 1.20E-08 | 1.74 |
| 268.19 | 589.52 | 0.01 | 6.56E-04 | 6.21E-03 | 1.53 |
| 342.03 | 625.52 | 0.03 | 8.91E-09 | 3.81E-07 | 1.90 |
| 324.0459 | 612.39 | 0.03 | 2.24E-09 | 1.56E-07 | 1.93 |
| 324.0457 | 652.06 | 0.02 | 6.59E-09 | 3.13E-07 | 2.57 |
| 307.02 | 607.78 | 0.03 | 2.10E-09 | 1.56E-07 | 1.74 |

### 2.5.4 Receiver operating characteristic curve (ROC)

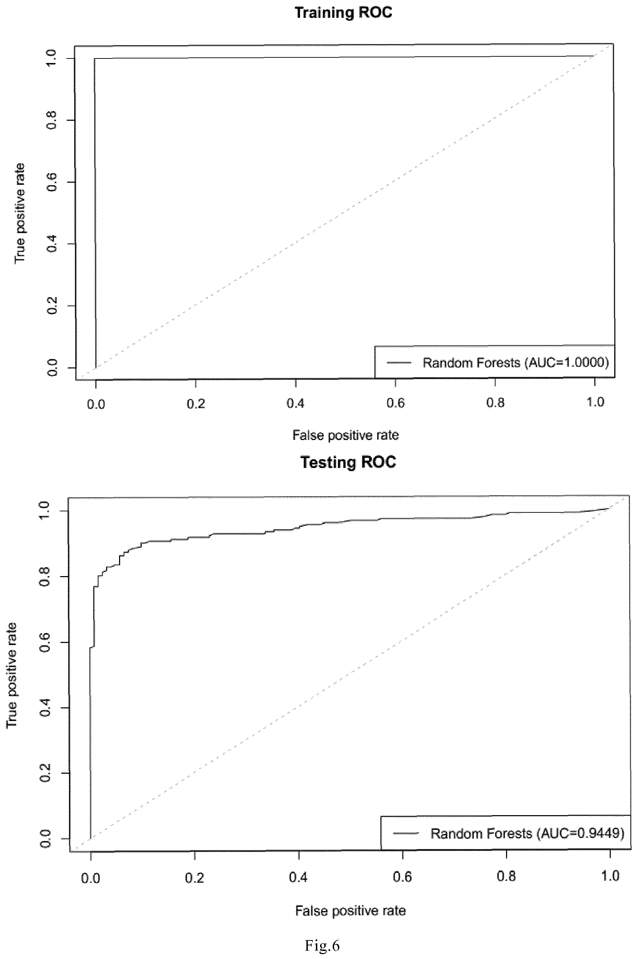

The eight potential markers were discriminated in the normal group and coronary heart disease group by using a random forest model (Random Forest)^{[7]} and receiver operating characteristic curve (ROC)^{[8]}. The data of peak areas of 92 metabolite profiles of the normal group and the coronary heart disease group were selected and used as training set via ROC modeling (see references [7] and [8]) (Table 2). In addition, 303 test samples (including 182 coronary heart disease samples and 121 normal control samples) were selected as test set. The test results showed AUC = 0.9449, FN (false negative) = 0.230, FP (false positive) = 0.008 (Fig.6). Thus, the present invention has high accuracy and specificity, and has good prospects to be developed as a diagnosis method to provide a basis for diagnosis of coronary heart disease.

**Table 2: Data of training set metabolite profiles (peak area)**

| Sample No. | Group (1: Coronary heart disease group; 0: normal group) | Mass-to-charge ratio (amu) | | | | | | | |
|---|---|---|---|---|---|---|---|---|---|
| | | 356.0722 | 284.1856 | 445.0662 | 268.191 | 342.0378 | 324.0459 | 324.0457 | 307.02 |
| N165_11_10 | 0 | 0.050727 | 0 | 0.0081 | 0.000576 | 0 | 0.007244 | 0 | 0 |
| N167_14_13 | 0 | 0.700671 | 0.491373 | 0.43858 | 0.258349 | 0.583474 | 1.01587 | 0.709247 | 0.996549 |
| N168_6_6 | 0 | 0.017057 | 0.003273 | 0.022923 | 0.000506 | 0 | 0 | 0 | 0 |
| N170_5_5 | 0 | 1.118726 | 0.763688 | 1.036212 | 0.587642 | 1.935456 | 1.544139 | 1.438488 | 1.665617 |
| N171_10_9 | 0 | 0.585286 | 0.399349 | 0.195601 | 0.257848 | 0.771351 | 0.918791 | 0.759376 | 0.763014 |
| N185_3_3 | 0 | 0.001756 | 0.002489 | 0.04602 | 0.001706 | 0.000674 | 0.001871 | 0.000765 | 0.004254 |
| N186_2_2 | 0 | 0.033602 | 0.002031 | 0 | 0.000286 | 0.018214 | 0 | 0 | 0 |
| N187_1_1 | 0 | 0.083965 | 0.018984 | 0.078802 | 0.024106 | 0.162598 | 0.231746 | 0.100976 | 0.214191 |
| N190_2_2 | 0 | 0.055174 | 0.025052 | 0.011355 | 0.017505 | 0.045384 | 0.058801 | 0.040412 | 0.050481 |
| N191_2_2 | 0 | 0.014361 | 0.000419 | 0 | 0 | 0.003741 | 0 | 0 | 0.026598 |
| N195_2_2 | 0 | 0.071606 | 0.072388 | 0.065478 | 0.03979 | 0.096294 | 0.130355 | 0.07972 | 0.095388 |
| N197_13_12 | 0 | 0.113316 | 0.126297 | 0.133139 | 0.091836 | 0.153965 | 0.15097 | 0.123104 | 0.230104 |
| N198_1_1 | 0 | 0.13997 | 0.154741 | 0.118181 | 0.133976 | 0.386868 | 0.450153 | 0.311324 | 0.448939 |
| N199_13_12 | 0 | 0.007775 | 0.018247 | 0.016699 | 0.010208 | 0.052443 | 0.006032 | 0.020938 | 0.06164 |
| N200_2_2 | 0 | 0.014128 | 0.055363 | 0.002909 | 0.015098 | 0.00202 | 0 | 0.001985 | 0.01716 |
| N201_2_2 | 0 | 0.014985 | 0.005288 | 0.005743 | 0.013153 | 0.004488 | 0.00455 | 0.007382 | 0.086503 |
| N203_13_12 | 0 | 0.114562 | 0.000885 | 0 | 0.007605 | 0 | 0 | 0.003256 | 0.057085 |
| N204_1_1 | 0 | 0.051158 | 0.082626 | 0.090507 | 0.054647 | 0.049881 | 0.059178 | 0.035429 | 0.059979 |
| N205_1_1 | 0 | 0.009121 | 0.00669 | 0 | 0.004913 | 0.003084 | 0 | 0.008396 | 0.012356 |
| N206_2_2 | 0 | 0.423082 | 0.000549 | 0.237519 | 0 | 0.388775 | 1.006219 | 0.538118 | 0.283513 |
| N207_2_2 | 0 | 0.037572 | 0.009893 | 0 | 0.00414 | 0.002972 | 0 | 0 | 0.007073 |
| N208_1_1 | 0 | 0.031229 | 0.031697 | 0 | 0.039781 | 0.010892 | 0 | 0.00776 | 0.004047 |
| N209_2_2 | 0 | 0.056193 | 0.025581 | 0.00882 | 0.01507 | 0.007239 | 0 | 0.008259 | 0.011088 |
| N212_1_1 | 0 | 0 | 0 | 0.006792 | 0.000325 | 0 | 0 | 0 | 0.021432 |
| N213_1_1 | 0 | 0.022905 | 0.003433 | 0 | 0.000721 | 0 | 0 | 0.003124 | 0 |
| N214_3_3 | 0 | 0.005787 | 0.000134 | 0.013291 | 0.00015 | 0 | 0 | 0.000927 | 0.011216 |
| N215_2_2 | 0 | 0.009559 | 0.001665 | 0 | 0.000675 | 0 | 0.003649 | 0.003024 | 0.003294 |
| N217_2_2 | 0 | 0.021546 | 0.012008 | 0 | 0.002519 | 0.010761 | 0.014342 | 0.005523 | 0.004285 |
| N218_1_1 | 0 | 0.001903 | 0.00647 | 0.002823 | 0.003972 | 0.001288 | 0 | 0 | 0.002533 |
| N220_3_3 | 0 | 0.037769 | 0.000147 | 0.021874 | 0.000497 | 0.061861 | 0.186411 | 0.096903 | 0.100274 |
| N222_1_1 | 0 | 0.031056 | 0.006934 | 0.084298 | 0.013115 | 0.087239 | 0.115484 | 0.071167 | 0.09323 |
| N223_1_1 | 0 | 0.022325 | 0.001591 | 0.005429 | 0.001879 | 0 | 0 | 0 | 0 |
| N226_2_2 | 0 | 0.401971 | 0.290546 | 0.328433 | 0.201518 | 0.682477 | 0.427509 | 0.552805 | 0.669991 |
| N227_2_2 | 0 | 0.014787 | 0.007046 | 0 | 0.005274 | 0.013257 | 0.002112 | 0.011434 | 0 |
| N228_5_5 | 0 | 0.228112 | 0.540553 | 0.195533 | 0.168774 | 0.430204 | 0.322248 | 0.331633 | 0.503222 |
| N229_6_6 | 0 | 0.141694 | 0.108248 | 0.083678 | 0.119914 | 0.383583 | 0.371522 | 0.212677 | 0.300032 |
| N231_9_8 | 0 | 0.379711 | 0.360892 | 0.166288 | 0.206992 | 0.108167 | 0.136906 | 0.092083 | 0.119944 |
| N232_6_6 | 0 | 0.047573 | 0.004747 | 0 | 0.000647 | 0.002274 | 0.00174 | 0 | 0 |
| N233_5_5 | 0 | 0.040641 | 0.080729 | 0.0423 | 0.053298 | 0.050616 | 0.127117 | 0.07407 | 0.062576 |
| N234_4_4 | 0 | 0.207284 | 0.200893 | 0.235156 | 0.150836 | 0.465188 | 0.399302 | 0.358934 | 0.341159 |
| N235_6_6 | 0 | 0.008056 | 0.049746 | 0.006451 | 0.067002 | 0.020476 | 0.002431 | 0.005861 | 0.009006 |
| N236_5_5 | 0 | 0.009005 | 0.000991 | 0 | 0.000181 | 0 | 0 | 0 | 0 |
| N237_4_4 | 0 | 0.055985 | 0.016887 | 0.004479 | 0.0146 | 0.053198 | 0.06221 | 0.059575 | 0.040373 |
| N238_4_4 | 0 | 1.900712 | 0.028984 | 1.165815 | 0.018644 | 1.571225 | 0.475648 | 1.529518 | 1.443399 |
| N239_4_4 | 0 | 0.072683 | 0.136582 | 0.141065 | 0.120443 | 0.390014 | 0.499418 | 0.188908 | 0.214719 |
| N241_4_4 | 0 | 0.004649 | 0.00051 | 0.038894 | 0 | 0 | 0 | 0 | 0.004573 |
| N242_3_3 | 0 | 0.006202 | 0.005425 | 0.018052 | 0.007253 | 0 | 0 | 0.001196 | 0.012753 |
| N243_5_5 | 0 | 0.21151 | 0.120443 | 0.229312 | 0.138598 | 0.476139 | 0.594607 | 0.389633 | 0.485419 |
| N244_14_13 | 0 | 0.013491 | 0.000969 | 0.004027 | 0.00156 | 0 | 0 | 0.001553 | 0.065665 |
| N245_5_5 | 0 | 0.076173 | 0.010746 | 0.002663 | 0.004756 | 0.008524 | 0.005277 | 0.003782 | 0.010315 |
| N247_6_6 | 0 | 0.00508 | 0.001569 | 0 | 0.0019 | 0.001161 | 0.007967 | 0.000846 | 0.037039 |
| N248_5_5 | 0 | 0.032339 | 0.000587 | 0 | 0.000437 | 0 | 0.008133 | 0 | 0 |
| ZSL229_2_2 | 1 | 1.018531 | 3.950757 | 0.182069 | 0.636612 | 0.60125 | 1.097666 | 0.289254 | 0.494088 |
| ZSL234_1_1 | 1 | 0.583531 | 3.435453 | 0.795557 | 1.709356 | 0.222236 | 0.761682 | 0.345999 | 0.187897 |
| ZSL235_2_2 | 1 | 1.181361 | 0.152603 | 0.939668 | 0.047144 | 0.929618 | 4.035717 | 1.292228 | 1.156159 |
| ZSL236_3_3 | 1 | 2.081281 | 0.018304 | 1.898479 | 0.006197 | 1.762673 | 1.136454 | 1.725205 | 1.479569 |
| ZSL237_4_4 | 1 | 6.492563 | 0.006244 | 14.87724 | 0.007235 | 22.19462 | 24.85611 | 17.40065 | 10.64721 |
| ZSL238_3_3 | 1 | 1.702545 | 11.98425 | 1.222842 | 7.900273 | 5.012054 | 0.637683 | 2.124289 | 3.730898 |
| ZSL239_6_6 | 1 | 2.162367 | 0.003073 | 4.745232 | 0.003427 | 5.442023 | 3.975631 | 3.293823 | 6.143599 |
| ZSL240_6_6 | 1 | 0.16421 | 0 | 0.047093 | 0.002727 | 0.044366 | 0.156119 | 0.049045 | 0.060174 |
| ZSL248_5_5 | 1 | 1.657123 | 6.083406 | 1.777601 | 2.504562 | 2.333015 | 4.393965 | 1.966348 | 1.972406 |
| ZSL250_5_5 | 1 | 8.714595 | 14.32087 | 26.98067 | 20.88803 | 15.94406 | 18.32642 | 13.07876 | 13.96109 |
| ZSL252_6_6 | 1 | 3.031666 | 0.008082 | 7.529829 | 0 | 7.073493 | 7.170141 | 2.691871 | 5.640622 |
| ZSL261_6_6 | 1 | 6.014641 | 0.189933 | 12.72496 | 13.93657 | 13.44713 | 32.8584 | 16.86183 | 10.9035 |
| ZSL265_6_6 | 1 | 11.22025 | 17.09913 | 18.15472 | 8.728776 | 9.174703 | 16.91267 | 20.83132 | 14.80546 |
| ZSL266_14_13 | 1 | 0.115196 | 0.088163 | 0.008972 | 0.110673 | 0.136602 | 0.0244 | 0.030063 | 0.059366 |
| ZSL267_5_5 | 1 | 1.49161 | 4.122593 | 2.400876 | 0.411089 | 1.302119 | 1.000125 | 1.343585 | 0.973993 |
| ZSL270_5_5 | 1 | 3.004328 | 0.013414 | 4.431113 | 0.003272 | 6.364588 | 10.60903 | 7.162436 | 9.64398 |
| ZSL271_5_5 | 1 | 4.564883 | 0.025178 | 2.33377 | 0.00848 | 5.587718 | 16.44589 | 6.301333 | 5.033009 |
| ZSL272_6_6 | 1 | 1.104237 | 11.81819 | 0.295694 | 20.48261 | 0.650639 | 0.350425 | 0.395211 | 0.463962 |
| ZSL277_6_6 | 1 | 2.611821 | 0.00141 | 2.03553 | 0.000691 | 8.739621 | 7.052004 | 3.151162 | 5.215719 |
| ZSL282_7_7 | 1 | 5.090599 | 0.00577 | 16.35812 | 0.000275 | 6.49348 | 20.55782 | 8.213584 | 7.685243 |
| ZSL289_7_7 | 1 | 7.652603 | 0.003049 | 8.715061 | 0.011397 | 18.01092 | 26.7367 | 13.21022 | 11.30195 |
| ZSL290_14_13 | 1 | 0.996549 | 4.390615 | 1.084703 | 6.741163 | 2.629733 | 3.284678 | 3.628341 | 3.778025 |
| ZSL297_6_6 | 1 | 1.066514 | 5.967553 | 0.722559 | 2.93087 | 0.664882 | 0.223668 | 0.204465 | 0.333787 |
| ZSL300_6_6 | 1 | 3.643793 | 1.006024 | 16.94684 | 0.148682 | 16.86828 | 4.310987 | 12.21613 | 12.20316 |
| ZSL301_6_6 | 1 | 0.199107 | 0.034298 | 0.143035 | 0.024563 | 0.098331 | 0.061123 | 0.045024 | 0.103692 |
| ZSL302_5_5 | 1 | 5.924905 | 5.237627 | 2.289743 | 5.691231 | 11.94612 | 20.6828 | 17.4122 | 14.4408 |
| ZSL312_5_5 | 1 | 0.035975 | 21.19713 | 0.00893 | 9.281833 | 0 | 0 | 0 | 0.005924 |
| ZSL314_6_6 | 1 | 0.555908 | 3.59192 | 0.177668 | 0.604776 | 0.264665 | 0.00398 | 0.11581 | 0.17875 |
| ZSL315_6_6 | 1 | 1.680925 | 3.099505 | 1.479545 | 5.030586 | 2.667323 | 4.141096 | 1.662493 | 2.903288 |
| ZSL317_14_13 | 1 | 3.527125 | 0.012436 | 3.266543 | 0.001539 | 6.087814 | 13.33405 | 6.072355 | 7.235422 |
| ZSL318_6_6 | 1 | 0.675975 | 3.398881 | 0.798803 | 4.353942 | 1.998757 | 5.442052 | 2.292455 | 2.906209 |
| ZSL330_14_13 | 1 | 1.011196 | 0.059638 | 1.455093 | 0.143674 | 0.274329 | 0.69689 | 0.947892 | 0.564808 |
| ZSL332_5_5 | 1 | 12.04706 | 9.802246 | 30.04653 | 6.986533 | 18.30787 | 24.86612 | 24.13673 | 17.45268 |
| ZSL334_6_6 | 1 | 0.116528 | 4.099484 | 0.038631 | 0.109829 | 0.005093 | 0 | 0 | 0 |
| ZSL335_6_6 | 1 | 0.025725 | 0.001383 | 0.038073 | 0 | 0 | 0 | 0.001766 | 0 |
| ZSL336_7_7 | 1 | 3.40415 | 0.022602 | 3.726221 | 0 | 17.21046 | 17.30863 | 10.59224 | 9.995526 |
| ZSL338_14_13 | 1 | 2.714739 | 3.426387 | 9.516216 | 3.730436 | 2.373802 | 7.024976 | 2.591477 | 3.802353 |
| ZSL340_13_12 | 1 | 3.615215 | 16.72375 | 4.245612 | 6.137015 | 5.698663 | 9.143185 | 7.359719 | 12.57524 |
| ZSL349_7_7 | 1 | 0.655089 | 2.645769 | 0.133089 | 1.350477 | 0.096092 | 0 | 0.315811 | 0.152866 |
| ZSL353_4_4 | 1 | 2.460175 | 8.463638 | 2.60743 | 7.497369 | 2.834242 | 6.970485 | 2.392618 | 3.338898 |

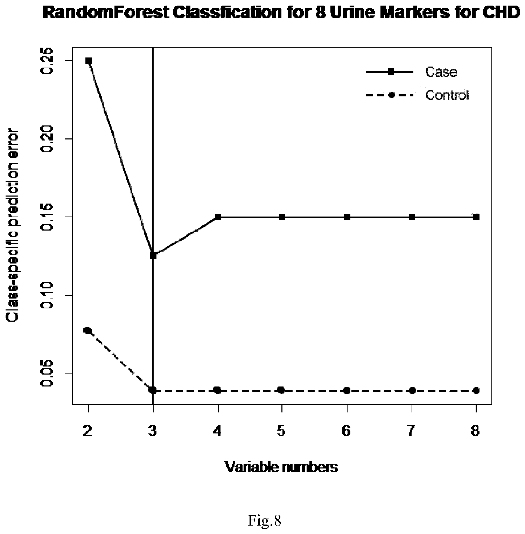

Using the random forest model to calculate the classification ability of the eight potential biomarkers for the obese group and the normal group, the results of the sorting ability (arranged from high to low) were shown in Table 3. The markers in the table should be tested using at least above 3 markers (Fig.8), so that the AUC value was around 0.90 while maintaining high sensitivity and specificity.

**Table 3: Classification ability of potential biomarkers**

| Metabolite (mass-to-charge ratio) (amu) | Interpreting value of normal group | Interpreting value of obese group | Mean Decrease Accuracy | Mean Decrease Gini |
|---|---|---|---|---|
| 356.07 | 0.150464 | 0.092498 | 0.121952 | 10.00917 |
| 445.06 | 0.104776 | 0.057948 | 0.082715 | 7.127041 |
| 284.18 | 0.080873 | 0.036778 | 0.06133 | 5.158795 |
| 324.0457 | 0.064424 | 0.043364 | 0.053989 | 6.110113 |
| 324.0459 | 0.055228 | 0.024179 | 0.041406 | 4.087854 |
| 342.03 | 0.052123 | 0.024192 | 0.039909 | 4.614609 |
| 268.19 | 0.068445 | 0.020407 | 0.045933 | 3.959031 |
| 307.02 | 0.033325 | 0.012134 | 0.024432 | 3.667505 |

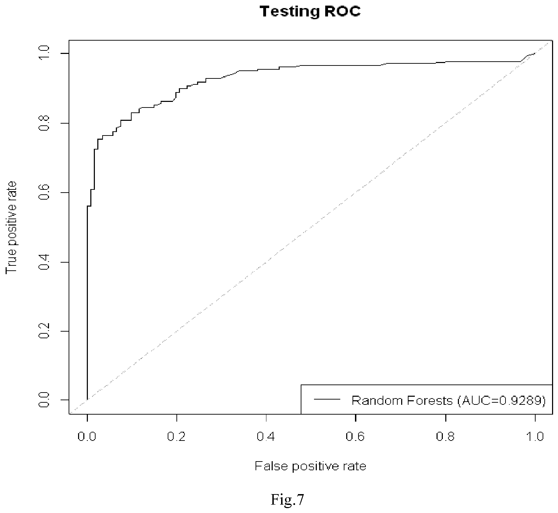

If mass-to-charge ratios, such as 356.07 and 445.06, were randomly removed from the training set, the resultant ROC test set (the above 303 test set samples) had AUC = 0.9289, AUC decreased significantly, FN = 0.296 and FP = 0.016, FN and FP significantly increased (Fig.7), which indicated the ability for diagnosis of coronary heart disease decreased.

### References:

[1] Finegold, JA; Asaria, P; Francis, DP. Mortality from ischaemic heart disease by country, region, and age: Statistics from World Health Organisation and United Nations. International journal of cardiology. 4 December 2012, 168 (2): 934-45.
[2] World Health Organization Department of Health Statistics and Informatics in the Information, Evidence and Research Cluster. The global burden of disease 2004 update. Geneva: WHO. 2004. ISBN 92-4-156371-0.
[3] Elizabeth Barrett-Connor. Gender differences and disparities in all-cause and coronary heart disease mortality: epidemiological aspects. Best Pract Res Clin Endocrinol Metab. 2013 Aug;27(4):481-500.
[4] Madjid M, Willerson JT. Inflammatory markers in coronary heart disease. Br Med Bull. 2011;100:23-38. doi: 10.1093/bmb/ldr043. Epub 2011 Oct 18.
[5] Spoletini I1, Vitale C, Rosano GM. Biomarkers for predicting postmenopausal coronary heart disease. Biomark Med. 2011 Aug;5(4):485-95. doi: 10.2217/bmm.11.51.
[6] Kishore Kumar Pasikanti, Kesavan Esuvaranathan, Paul C. Ho, et al. Noninvasive urinary metabonomic diagnosis of human bladder cancer. Journal of Proteome Research, 2010, 9, 2988-2995.
[7] Liaw, Andy & Wiener, Matthew. Classification and Regression by randomForest, R News (2002), Vol. 2/3 p. 18.
[8] Jianguo Xia, David I. Broadhurst, Michael Wilson, David S. Wishart. Translational biomarker discovery in clinical metabolomics: an introductory tutorial. Metabolomics (2013) 9:280-299.

## Claims

1. A method for risk assessment, diagnosis, early diagnosis or pathological staging of coronary heart disease, comprising a step of determining content of each biomarker of a biomarker composition in a urine sample of a subject;
wherein, the biomarker composition comprises at least Biomarkers 1 to 3 of the following Biomarkers 1 to 8:
Biomarker 1, which has a mass-to-charge ratio of 356.07 ± 0.4amu, and a retention time of 606.57 ± 60s;
Biomarker 2, which has a mass-to-charge ratio of 284.18 ± 0.4amu, and a retention time of 538.89 ± 60s;
Biomarker 3, which has a mass-to-charge ratio of 445.06 ± 0.4amu, and a retention time of 494.89 ± 60s;
Biomarker 4, which has a mass-to-charge ratio of 268.19 ± 0.4amu, and a retention time of 589.52 ± 60s;
Biomarker 5, which has a mass-to-charge ratio of 342.03 ± 0.4amu, and a retention time of 625.52 ± 60s;
Biomarker 6, which has a mass-to-charge ratio of 324.0459 ± 0.4amu, and a retention time of 612.39 ± 60s;
Biomarker 7, which has a mass-to-charge ratio of 324.0457 ± 0.4amu, and a retention time of 652.06 ± 60s; and
Biomarker 8, which has a mass-to-charge ratio of 307.02 ± 0.4amu, and a retention time of 607.78 ± 60s;
wherein a liquid chromatography-mass spectrometry method is used for determining content of each biomarker of the biomarker composition in a urine sample of a subject;
wherein a C18 column and an elution solvent of 0.1% (v/v) formic acid in acetonitrile solution s used in the liquid chromatography-mass spectrometry method;
wherein chromatographic conditions are as follows:
Column: C18 column; mobile phase A: 0.1% formic acid aqueous solution, mobile phase B: 0.1% formic acid in acetonitrile solution; gradient elution program: 0 ∼ 3 min, 5% B, 3 ∼ 36 min, 5% ∼ 80% B, 36 ∼ 40 min, 80% ∼ 100% B, 40 ∼ 45 min, 100% B, 45 ∼ 50 min, 100% ∼ 5% B, 50 ∼ 60 min, 5% B; flow rate: 0.2 mL / min; injection volume: 20 µL.

2. The method according to claim 1, wherein the biomarker composition further comprises one or more of Biomarkers 4 to 8.

3. The method according to claim 1, wherein the biomarker composition comprises Biomarkers 1 to 8.

4. The method according to claim 1, wherein the method further comprises a step of establishing a training set for contents of the biomarker composition according to claim 1 in a urine samples of a coronary heart disease subject and a normal subject.

5. The method according to claim 4, wherein the training set is established by using a multivariate statistical classification model.

6. The method according to claim 5, wherein said multivariate statistical classification model is a random forest model.

7. The method according to claim 5 or claim 6, wherein the training set comprises data as shown in Table 2.

8. The method according to claim 1, wherein the method further comprises a step of comparing the content of each biomarker of the biomarker composition in a urine sample of a subject to data of a training set, and the training set is for contents of the biomarker composition according to claim 1 in samples of a coronary heart disease subject and a normal subject.

9. The method according to claim 8, wherein the training set is established by using a multivariate statistical classification model.

10. The method according to claim 9, wherein said multivariate statistical classification model is a random forest model.

11. The method according to claim 9 or claim 10, wherein the training set comprises data as shown in Table 2.

12. The method according to any one of claims 8 to 11, wherein the step of comparing the content of each biomarker is carried out by using a receiver operating characteristic curve.

13. The method according to claim 12, wherein the result from the step of comparing the content of each biomarker is interpreted by a method comprising: if a subject is assumed to be a non-coronary heart disease subject, and his probability of non-coronary heart disease diagnosed by ROC is less than 0.5 or his probability of coronary heart disease diagnosed by ROC is greater than 0.5, the subject is determined to have a high probability or a higher risk of coronary heart disease, or is diagnosed as a patient with coronary heart disease.

## Patentansprüche

1. Verfahren zur Risikoeinschätzung, Diagnose, Früherkennung oder pathologischen Stadieneinteilung von koronarer Herzerkrankung, umfassend einen Schritt des Bestimmens des Gehalts jeden Biomarkers einer Biomarkerzusammensetzung in einer Urinprobe eines Individuums;
wobei die Biomarkerzusammensetzung mindestens die Biomarker 1 bis 3 der folgenden Biomarker 1 bis 8 umfasst:
Biomarker 1, der ein Masse-zu-Ladung-Verhältnis von 356,07 ± 0,4 amu und eine Retentionszeit von 606,57 ± 60 s aufweist;
Biomarker 2, der ein Masse-zu-Ladung-Verhältnis von 284,18 ± 0,4 amu und eine Retentionszeit von 538,89 ± 60 s aufweist;
Biomarker 3, der ein Masse-zu-Ladung-Verhältnis von 445,06 ± 0,4 amu und eine Retentionszeit von 494,89 ± 60 s aufweist;
Biomarker 4, der ein Masse-zu-Ladung-Verhältnis von 268,19 ± 0,4 amu und eine Retentionszeit von 589,52 ± 60 s aufweist;
Biomarker 5, der ein Masse-zu-Ladung-Verhältnis von 342,03 ± 0,4 amu und eine Retentionszeit von 625,52 ± 60 s aufweist;
Biomarker 6, der ein Masse-zu-Ladung-Verhältnis von 324,0459 ± 0,4 amu und eine Retentionszeit von 612,39 ± 60 s aufweist;
Biomarker 7, der ein Masse-zu-Ladung-Verhältnis von 324,0457 ± 0,4 amu und eine Retentionszeit von 652,06 ± 60 s aufweist; und
Biomarker 8, der ein Masse-zu-Ladung-Verhältnis von 307,02 ± 0,4 amu und eine Retentionszeit von 607,78 ± 60 s aufweist;
wobei ein Flüssigchromatografie-Massenspektrometrie-Verfahren benutzt wird, um den Gehalt jeden Biomarkers der Biomarkerzusammensetzung in einer Urinprobe eines Individuums zu bestimmen;
wobei eine C18-Säule und ein Elutionsmittel von 0,1 % (v/v) Ameisensäure in Acetonitrillösung in dem Flüssigchromatografie-Massenspektrometrie-Verfahren benutzt wird;
wobei die Chromatografiebedingungen folgende sind:
Säule: C18-Säule; mobile Phase A: 0,1 % Ameisensäure, wässrige Lösung; mobile Phase B: 0,1 % Ameisensäure in Acetonitrillösung; Gradientenelutionsprogramm: 0 bis 3 Min., 5 % B, 3 bis 36 Min., 5 % bis 80 % B, 36 bis 40 Min., 80 % bis 100 % B, 40 bis 45 Min., 100 % B, 45 bis 50 Min., 100 % bis 5 % B, 50 bis 60 Min., 5 % B; Flussrate: 0,2 ml/Min.; Injektionsvolumen: 20 µl.

2. Verfahren nach Anspruch 1, wobei die Biomarkerzusammensetzung weiterhin einen oder mehrere der Biomarker 4 bis 8 umfasst.

3. Verfahren nach Anspruch 1, wobei die Biomarkerzusammensetzung die Biomarker 1 bis 8 umfasst.

4. Verfahren nach Anspruch 1, wobei das Verfahren weiterhin einen Schritt des Einrichtens eines Trainingssatzes für den Gehalt der Biomarkerzusammensetzung nach Anspruch 1 in einer Urinprobe eines Individuums mit koronarer Herzerkrankung und eines unauffälligen Individuums umfasst.

5. Verfahren nach Anspruch 4, wobei der Trainingssatz eingerichtet wird, indem ein multivariates statistisches Klassifikationsmodell benutzt wird.

6. Verfahren nach Anspruch 5, wobei das multivariate statistische Klassifikationsmodell ein Random-Forest-Modell ist.

7. Verfahren nach Anspruch 5 oder 6, wobei der Trainingssatz die Daten, die in Tabelle 2 gezeigt werden, umfasst.

8. Verfahren nach Anspruch 1, wobei das Verfahren weiterhin einen Schritt des Vergleichens des Gehalts jeden Biomarkers der Biomarkerzusammensetzung in einer Urinprobe eines Individuums mit den Daten eines Trainingssatzes umfasst, und wobei der Trainingssatz den Gehalt der Biomarkerzusammensetzung nach Anspruch 1 in Proben eines Individuum mit koronarer Herzerkrankung und eines unauffälligen Individuums umfasst.

9. Verfahren nach Anspruch 8, wobei der Trainingssatz eingerichtet wird, indem ein multivariates statistisches Klassifikationsmodell benutzt wird.

10. Verfahren nach Anspruch 9, wobei das multivariate statistische Klassifikationsmodell ein Random-Forest-Modell ist.

11. Verfahren nach Anspruch 9 oder 10, wobei der Trainingssatz die Daten, die in Tabelle 2 gezeigt werden, umfasst.

12. Verfahren nach einem der Ansprüche 8 bis 11, wobei ein Schritt des Vergleichens des Gehalts jeden Biomarkers durchgeführt wird, indem eine ROC-Kurve benutzt wird.

13. Verfahren nach Anspruch 12, wobei das Ergebnis aus dem Schritt des Vergleichens des Gehalts jeden Biomarkers durch ein Verfahren interpretiert wird, umfassend: Wenn angenommen wird, dass ein Individuum ein Individuum mit nicht-koronarer Herzerkrankung ist, und seine Wahrscheinlichkeit einer durch ROC diagnostizierten nicht-koronaren Herzerkrankung weniger als 0,5 beträgt, oder seine Wahrscheinlichkeit einer durch ROC diagnostizierten koronaren Herzerkrankung mehr als 0,5 beträgt, wird bestimmt, dass das Individuum eine hohe Wahrscheinlichkeit oder ein höheres Risiko für eine koronare Herzerkrankung aufweist, oder es wird als ein Patient mit einer koronaren Herzerkrankung diagnostiziert.

## Revendications

1. Procédé d'évaluation des risques, de diagnostic, de diagnostic précoce ou de stadification pathologique des maladies cardiaques coronariennes, comprenant une étape consistant à déterminer la quantité de chaque biomarqueur d'une composition de biomarqueur dans un échantillon d'urine d'un sujet ;
dans lequel, la composition de biomarqueur comprend au moins les biomarqueurs 1 à 3 parmi les marqueurs 1 à 8 suivants :
Biomarqueur 1, qui a un rapport masse sur charge de 356,07 ± 0,4 amu et un temps de rétention de 606,57 ± 60 s ;
Biomarqueur 2, qui a un rapport masse sur charge de 284,18 ± 0,4 amu et un temps de rétention de 538,89 ± 60 s ;
Biomarqueur 3, qui a un rapport masse sur charge de 445, 06 ± 0,4 amu et un temps de rétention de 494,89 ± 60 s ;
Biomarqueur 4, qui a un rapport masse sur charge de 268,19 ± 0,4 amu et un temps de rétention de 589,52 ± 60 s ;
Biomarqueur 5, qui a un rapport masse sur charge de 342,03 ± 0,4 amu et un temps de rétention de 625,52 ± 60 s ;
Biomarqueur 6, qui a un rapport masse sur charge de 324,0459 ± 0,4 amu et un temps de rétention de 612,39 ± 60 s ;
Biomarqueur 7, qui a un rapport masse sur charge de 324,0457 ± 0,4 amu, et un temps de rétention de 652,06 ± 60 s ; et
Biomarqueur 8, qui a un rapport masse sur charge de 307,02 ± 0,4 amu et un temps de rétention de 607,78 ± 60 s ;
dans lequel un procédé de chromatographie liquide couplée à un spectromètre de masse est utilisé pour la détermination de la quantité de chaque biomarqueur dans la composition de biomarqueur dans un échantillon d'urine d'un sujet ;
dans lequel une colonne C18 et un solvant d'élution à base d'acide formique à 0,1 % (en volume) dans une solution d'acétonitrile est utilisé dans le procédé de chromatographie liquide couplée à un spectromètre de masse ;
dans lequel les conditions chromatographiques sont les suivantes :
Colonne : colonne C18 ; phase mobile A : solution aqueuse d'acide formique à 0,1 %, phase mobile B : acide formique à 0,1 % dans une solution d'acétonitrile ; programme d'élution du gradient : de 0 à 3 min, 5 % de B, de 3 à 36 min, entre 5 % et 80 % de B, de 36 à 40 min, entre 80 % et 100 % de B, de 40 à 45 min, 100 % de B, de 45 à 50 min, entre 100 % et 5 % de B, de 50 à 60 min, 5 % de B ; débit : 0,2 mL/min ; volume d'injection : 20 µL.

2. Procédé selon la revendication 1, dans lequel la composition de biomarqueur comprend en outre au moins un des biomarqueurs 4 à 8.

3. Procédé selon la revendication 1, dans lequel la composition de biomarqueur comprend en outre les biomarqueurs 1 à 8.

4. Procédé selon la revendication 1, dans lequel le procédé comprend en outre une étape d'établissement d'un ensemble d'entraînement pour des quantités de la composition de biomarqueur selon la revendication 1 dans un échantillon d'urine chez un sujet atteint d'une maladie cardiaque coronarienne et chez un sujet normal.

5. Procédé selon la revendication 4, dans lequel l'ensemble d'entraînement est établi en utilisant un modèle de classification statistique multivariée.

6. Procédé selon la revendication 5, dans lequel ledit modèle de classification statistique multivariée est un modèle arborescent aléatoire.

7. Procédé selon la revendication 5 ou la revendication 6, dans lequel l'ensemble d'entraînement comprend des données telles qu'indiquées dans le tableau 2.

8. Procédé selon la revendication 1, dans lequel le procédé comprend en outre une étape de comparaison de la quantité de chaque biomarqueur de la composition de biomarqueur dans un échantillon d'urine d'un sujet avec des données d'un ensemble d'entraînement, et l'ensemble d'entraînement est destiné à des quantités de la composition de biomarqueur selon la revendication 1 dans des échantillons d'un sujet atteint d'une maladie cardiaque coronarienne et d'un sujet normal.

9. Procédé selon la revendication 8, dans lequel l'ensemble d'entraînement est établi en utilisant un modèle de classification statistique multivariée.

10. Procédé selon la revendication 9, dans lequel ledit modèle de classification statistique multivariée est un modèle arborescent aléatoire.

11. Procédé selon la revendication 9 ou la revendication 10, dans lequel l'ensemble d'entraînement comprend des données telles qu'indiquées dans le tableau 2.

12. Procédé selon l'une quelconque des revendications 8 à 11, dans lequel l'étape de comparaison de la quantité de chaque biomarqueur est effectuée en utilisant une courbe caractéristique de fonctionnement du récepteur.

13. Procédé selon la revendication 12, dans lequel le résultat provenant de l'étape de comparaison de la quantité de chaque biomarqueur est interprété au moyen d'un procédé comprenant : si un sujet est supposé être un sujet atteint d'une maladie cardiaque non coronarienne, et sa probabilité de diagnostic de maladie cardiaque non coronarienne par ROC est inférieure à 0,5 ou sa probabilité de diagnostic de maladie cardiaque coronarienne par ROC est supérieure à 0,5, il a été déterminé que le sujet a une haute probabilité ou un risque élevé de maladie cardiaque coronarienne, ou est diagnostiqué en tant que patient atteint de maladie cardiaque coronarienne.
